# EUROPÄISCHE PATENTSCHRIFT

(11) **EP 1 888 474 B1**
(45) Veröffentlichungstag und Bekanntmachung des Hinweises auf die Patenterteilung: **06.04.2011**
(21) Anmeldenummer: 06763405.5
(22) Anmeldetag: 31.05.2006
(51) Int. Cl.: C03C 10/00, A61K 6/027, A61K 6/06

(54) **DENTALGLASKERAMIKEN**
DENTAL GLASS CERAMICS
VITROCERAMIQUES DENTAIRES

(30) Priorität: 08.06.2005 DE 102005026269
(43) Veröffentlichungstag der Anmeldung: 20.02.2008
(73) Patentinhaber: Ivoclar Vivadent AG, 9494 Schaan (LI)
(72) Erfinder: HÖLAND, Wolfram, CH-9494 Schaan (LI); RITZBERGER, Christian, A-6710 Nenzing (AT); RHEINBERGER, Volker, CH-9490 Vaduz (LI); APEL, Elke, CH- 9479 OBERSCHAN (CH)
(74) Vertreter: Fitzner, Uwe
(86) Internationale Anmeldenummer: PCT/EP2006/062761
(87) Internationale Veröffentlichungsnummer: WO 2006/131473

(56) Entgegenhaltungen:
- DE-A1- 10 346 197
- DE-A1- 10 351 885
- DE-A1- 19 725 552
- DE-C1- 4 423 794
- US-A- 5 948 516

## Beschreibung

Die Erfindung betrifft Dentalglaskeramiken sowie ein Verfahren zu deren Herstellung und deren Verwendung.

Nach dem Stand der Technik ist bekannt, dass Glaskeramiken für technische Anwendungen und für Dentalglaskeramiken durch ein Gefüge gekennzeichnet sind, das eine oder mehrere Kristallphasen oder eine oder mehrere Glasphasen enthält. Grundsätzlich ist vorauszuschicken, dass eine bekannte Methode zur Erzeugung eines derartigen Gefüges die gesteuerte Kristallisation von Ausgangsgläsern durch die Steuerung der Keimbildungs- und Kristallisationsprozesse im speziellen Ausgangsglas ist. Auf dieser Basis gibt es eine Vielzahl komplizierter Stoffsysteme, die als Ausgangsgläser für Dentalglaskeramiken dienen (W. Höland, G.H. Beall, Glass-ceramic technology 2002, The American Ceramic Society, Westerville, OH, USA). Für Dentalglaskeramiken besitzen die optischen Eigenschaften einen besonderen Stellenwert. Die herausragende Eigenschaft von Edelsteinen und Schmucksteinen ist ebenfalls ihre optische Brillanz. Daher wird zuerst der Stand der Technik analysiert, in welchem Mineralien mit Edel- oder Schmucksteincharakter in Glaskeramiken bekannt sind. Aus der oben zitierten Literaturstelle (Höland, Beall, 2002) sind z.B. Glaskeramiken für technische Anwendungen bekannt, die Kristalle vom Typ des Spinell, des Enstatit oder des Cordierit enthalten. Diese Kristallarten sind beispielsweise als Edel- oder Schmucksteine bekannt. Allerdings spielen in diesen Glaskeramiken für technische Anwendungen die optischen Eigenschaften keine Rolle.

Als Schmuckstein ist auch natürlicher Hydroxyapatit, Ca₅(PO₄)₃OH, bekannt. Aufgrund des relativ hohen OH-Gehaltes im natürlichen Apatit konnte dieser Kristall in Dentalglaskeramiken für die restaurative Zahnmedizin nicht gebildet werden. Für die Zwecke der Restauration von Zähnen wurden daher auf synthetischem Wege Fluorapatit, Ca₅(PO₄)₃F, in Glaskeramiken entwickelt.

Der Xenotim-Kristall, YPO₄, besitzt auch Schmucksteincharakter. Produkte mit Xenotim-Kristallen sind aus dem Stand der Technik bekannt (J. Am. Ceram. Soc. 81, 1998, 2216-2218). Solche Produkte finden beispielsweise in der Medizin Verwendung für die Tumorbehandlung (Kawashita et al: Biomaterials 24(2003) Heft 17, 2955 bis 2963). Die Verwendung in der Technik als keramische Komposite ist aus der US 5,948,516 bekannt.

In der DE 102 45 234 A1 werden Xenotim-haltige Glaskeramiken als Produkte mit hohem E-Modul beschrieben. Bei diesen Produkten handelt es sich um SiO₂-arme und Al₂O₃-reiche Glaskeramiken für technische Anwendungen. Hinsichtlich der optischen Eigenschaften enthält dieser Stand der Technik keinerlei Aussagen. Ähnliches gilt auch für die DE 103 46 197 A1.

Aufgabe der vorliegenden Erfindung ist es, eine Dentalglaskeramik zur Verfügung zu stellen, welche gegenüber dem Stand der Technik verbesserte optische Eigenschaften aufweist und insbesondere für Werkstoffe für die restaurative Zahnheilkunde einsetzbar ist. Ziel ist es, eine Lichttransmission (Lichtdurchlässigkeit), Lichtstreuung und Beugung zu erreichen, die derjenigen von natürlichen Zähnen entspricht. Ferner sollen Dentalglaskeramiken zur Verfügung gestellt werden, welche als Spezialkomponenten für Verblendwerkstoffe mit hoher optischer Ästhetik verwendet werden können. Hierdurch soll ermöglicht werden, Verblendungen zur Verfügung zu stellen.

Diese Aufgabe wird dadurch gelöst, daß die Dentalglaskeramik als Hauptkristallphase Edel- und/oder Schmucksteine, vorzugsweise Kristalle vom Xenotim-Typ oder Monazit-Typ oder Gemische hiervon enthält oder deren Mischkristalle oder deren verwandte Kristalle, wie z.B. die vom Rhabdophan-Typ enthält.

In den erfindungsgemässen Glaskeramiken werden bevorzugt Xenotim- und/oder Monazit-Kristalle bzw. Kristalle der Xenotim- und/oder Monazit-Typen wie YPO₄, ErPO₄, YbPO₄, LuPO₄, LaPO4, CePO4, DyPO₄, GdPO₄ oder TbPO₄ gebildet. Ebenso sind deren mineralogische Verwandten, wie vom Rhabdophan- und/oder Weinschenkit-Typ wie RPO₄ * (0,5-1 )Z bzw. RPO4 * 2Z einsetzbar, wobei R ein Element der seltenen Erden, wie z.B. Y, Er oder Dy darstellt und Z in Mineralien bevorzugt für H₂O steht.

In den erfindungsgemässen Glaskeramiken ist die Bildung von Xenotim oder verwandte Phasen bevorzugt. Das heißt, erfindungsgemäß enthält die Hauptkristallphase YPO₄ oder sie besteht aus dieser Verbindung. Ebenso können Gemische eines oder mehrerer der zuvor genannten weiteren Kristalle vom Xenotim-Typ, Monazit-Typ, deren Mischkristalle oder verwandte Kristalle , wie die vom Rhabdophan- Typ erfindungsgemäß gebildet werden. Nähere Ausführungen zu den Xenotimen und Monaziten sind z.B. in folgenden Literaturstellen enthalten:
Ushakov, S.V., Helean, K.B. and Navrotsky, A. "Thermochemistry of rare-earth orthophosphates, Journal Mater Res., Vol. 16, No. 9, 2623-2632 (Sept. 2001),
Inoue, M. Nakamura, T., Otsu, H., Kominami, H. and Inui T. Nippon Kagaku Kaishi 5, 612 (1993),
Hikichi, V., Yu, C.F., Miyamoto, V. and Okada, S.J. Alloys Compd. 192, 102 (1993) Hikichi et. al. J. Am.Ceram. Soc. 76, 1073-1076 (1989).

Die Hauptkristallphase kann durch gesteuerte Volumenkeimbildung und - kristallisation im Ausgangsglas erzeugt werden, so dass die Kristalle einzeln und im Volumen statistisch verteilt wachsen und ihre Größe im Bereich von 0,1 bis 10 µm, bevorzugt von 0,1 bis 2 µm, steuerbar ist.

Die Steuerung der Vorgänge des Kristallwachstums wird durch zwei Hauptwirkungen verursacht: Die Wahl der Zusammensetzung und die nachträgliche Wärmebehandlung der erschmolzenen Ausgangsgläser. Hierbei spielen sowohl die Temperatur als auch die Zeit wichtige Rollen. Eine Besonderheit der Wärmebehandlung ist, dass neben der einstufigen auch mehrstufige thermische Behandlungen zur Kristallisation führen. Ein Sonderfall stellt eine solche Glasschmelze dar, die an Ionen und Stoffgruppen für die Kristalle derart übersättigt ist, dass sie bereits bei Abkühlung, also ohne eine zusätzliche Wärmebehandlung, Kristalle bildet. Diese Kristallisation läuft spontan, also nicht kontrolliert ab, die Kristallgrösse ist hierbei nicht steuerbar. Eine Steuerung ist nur durch eine zusätzliche thermische Behandlung möglich.

Erfindungsgemäß können neben der Hauptkristallphase aus Xenotim auch weitere Sekundärkristallphasen in der Dentalglaskeramik vorhanden sein. Für solche Sekundärkristallphasen kommen, ausgehend von der Zusammensetzung der Ausgangsgläser, insbesondere Apatit oder Leucit in Betracht. Erfindungsgemäß ist es überraschend, daß die vorteilhaften Eigenschaften, insbesondere die optischen Eigenschaften, wie Transluzenz mit steuerbarer Opaleszenz und thermische Eigenschaften, wie variable Sintertemperaturen, welche durch die Hauptkristallphase aus Xenotim-Kristallen bedingt sind, auch in einem Mischglaskeramikgefüge mit den beschriebenen Sekundär kristallphasen aufrechterhalten bleiben.

Die beschriebenen Mischkristallkeramiken sind darüber hinaus kompatibel zu anderen Dentalglaskeramiken, z.B. Leucit-Dentalglaskeramiken oder Leucit-Apatit-Dentalglaskeramiken. Somit eröffnen sich weitere Anwendungsmöglichkeiten der erfindungsgemäßen Dentalglaskeramik zur Herstellung von Sinterglaskeramiken mit Leucit- und/oder Leucit-Apatit-Glaskeramik-Systemen.

Erfindungsgemäß sind die weiteren Gläser vorzugsweise ausgewählt aus der Gruppe der Opalgläser, der Alkalisilicatgläser und/oder bei niedriger Temperatur sinternden Kalium-Zink-Silicat-Gläser sowie die weiteren Glaskeramiken, vorzugsweise ausgewählt sind aus der Gruppe der tiefsinternden Apatitglaskeramiken, der transluzenten Apatitglaskeramiken, der sinterbaren Lithiumsilicatglaskeramiken, der ZrO₂-SiO₂- Glaskeramiken und/oder der Leucit enthaltenden Phosphosilicatglaskeramiken.

Die Xenotim-Typ-Glaskeramiken gemäß der Erfindung können mit anderen Gläsern und/oder Glaskeramiken gemischt werden. In diesem Falle bildet die Xenotim-Glaskeramik die Hauptkomponente in einem beispielsweise anorganischen Komposit. Die erfindungsgemässen Glaskeramiken können hierbei mit einer Vielzahl von anderen Glaskeramiken kombiniert werden, um die Einstellung bestimmter optischer, mechanischer oder thermischer Eigenschaften zu erreichen. Beispiele solcher Gläser oder Glaskeramiken sind z.B. zu finden in der DE 43 14 817 A1, DE 44 23 793 A1, DE 44 23 794 A1, DE 44 28 839 A1, DE 196 47 739 A1, DE 197 25 552 A1 und DE 100 31 431.A1 Solche Gläser und Glaskeramiken werden vorzugsweise von Silicaten, Boraten oder Phosphaten oder Aluminat-Silicat-Systemen hergeleitet. Bevorzugte Glaskeramiken werden aus folgenden Stoffsystemen abgeleitet: SiO₂-Al₂O₃-K₂O mit kubischen oder tetragonalen Leucitkristallen, SiO₂-B₂O₃-Na₂O, Alkali-Silicaten, Alkali-Zink-Silicaten, Silico-Phosphat- Systeme n und/oder dem SiO₂-ZrO₂ Grundsystem. Bei der Mischung solcher Gläser und/oder Glaskeramiken mit Xenotim-Glaskeramiken gemäß der Erfindung kann der thermische Expansionskoeffizient der daraus resultierenden Mischglaskeramiken in einem Bereich von 7 bis 20 * 10 - ⁶ K⁻¹, gemessen im Bererich von 100 bis 400 °C, eingestellt werden.

Darüber hinaus kann die erfindungsgemäße Dentalglaskeramik noch ein oder mehrere farbgebende oder fluoreszenzbildende Metalloxide enthalten. Hierfür kommen vorzugsweise Metalle aus den Nebengruppen 1 bis 8 mit den Seltenerd-Elementen des Periodensystems der Elemente in Betracht.

Die erfindungsgemässe Dentalglaskeramik enthält als Hauptkomponenten SiO₂, Al₂O₃, Y₂O₃ und P₂O₅. Als Zusatzkomponenten können z. B. Na₂O, K₂O, B₂O₃, Li₂O, ZrO₂, CeO₂, TiO₂, ZnO, CaO, La₂O₃, Tb₄O₇ oder Fluorid zum Einsatz kommen. Erfindungsgemäß kommen vorzugsweise Alkalioxide, insbesondere Na₂O oder K₂O, in Betracht.

Als allgemeine Zusammensetzung der erfindungsgemässen Dentalglaskeramik mit Xenotim- und/ oder Monazit- Kristallen und/oder Kristallen des Xenotim- oder Monazit-Typ- Kristallen kommt z.B. folgende in Betracht:

| Komponente | Ma.-% |
|---|---|
| SiO₂ | 40 - 65 |
| Al₂O₃ | 5 - 25 |
| R- Oxide | 0.5 - 30 |
| P₂O₅ | 0.5 - 10 |

wobei die R- Oxide ausgewählt sind aus der Gruppe Y₂O₃, CeO2, La₂O₃, Tb₄O₇, Er₂O₃ und/oder Lu₂O₃.

So besteht eine bevorzugte erfindungsgemässe Dentalglaskeramik aus

| Komponente | Ma.-% |
|---|---|
| SiO₂ | 45 - 60 |
| Al₂O₃ | 8 - 20 |
| Y₂O₃ | 5 - 30 |
| P₂O₅ | 1 - 10 |
| B₂O₃ | 0 - 10 |
| Na₂O | 0 - 15 |
| K₂O | 0 - 15 |
| Li₂O | 0 - 5 |
| CaO | 0 - 10 |
| Fluorid | 0 - 5 |

Die Summe der im Glas bzw. der Glaskeramik enthaltenen Komponenten beträgt dabei stets 100 Ma.-%.

Es wurde weiterhin herausgefunden, dass das Molverhältnis von P₂O₅ zu Y₂O₃ vorzugsweise im Bereich von 1 : 0,5 bis 1 : 3,5 liegen sollte. Für die Zusammensetzungen 1 bis 6 aus der Tabelle 2 sind in nachstehender Tabelle 1 die Molverhältnisse angegeben.

**Tabelle 1: Molverhältnisse P₂O₅:Y₂O₃**

| Chem. Komponente/Eigenschaft | 1 | 2 | 3 | 4 | 5 | 6 |
|---|---|---|---|---|---|---|
| P₂O₅ | 1.0 | 1.0 | 1.0 | 1.0 | 1.0 | 1.0 |
| Y₂O₃ | 1.0 | 1.58 | 3.15 | 1.58 | 1.59 | 2.0 |
| Kristallphase | YPO₄·* 0.8Z | YPO₄· 0.8Z und Fluorapatit | YPO₄ * 0.8 Z | YPO₄ * 0.8 Z | YPO4·* 0.8Z | YPO₄ |

Während Z in Mineralien bevorzugt für H₂O steht, bedeutet es in synthetischen Gläsern und Glaskeramiken Fremdionen oder Gitterfehlstellen.

Die beschriebenen erfindungsgemäßen Dentalglaskeramiken in ihren verschiedenen Zusammensetzungen zeichnen sich durch ausgezeichnete transluzente Eigenschaften mit einem kontrollierten Trübungsgrad aus, ohne daß ein vollständig weiß-opaker Körper ohne jede Transluzenz (entspricht einem Trübungsgrad von 1,0) entsteht. Der Trübungsgrad, gemessen nach der Dentalnorm BS 5612, sollte hierbei kleiner gleich 0,8 betragen und liegt vorzugsweise im Bereich von 0,40 bis 0,78.

Ferner haben die Dentalglaskeramiken gute Verarbeitungseigenschaften. Da sie auch mit weiteren Glaskeramiken gemischt werden können, lassen diese sich bevorzugt in der restaurativen Zahnheilkunde, insbesondere als sinterbarer Verblendwerkstoff verwenden.

Vorteilhaft ist auch die gute chemische Beständigkeit der erfindungsgemäßen Dentalglaskeramiken. Diese zeigt sich insbesondere in einer ausgezeichneten Säurebeständigkeit, die vorzugsweise weniger als 100 µg/cm² Masseverlust beträgt.

Die erfindungsgemäßen Dentalglaskeramiken können mit üblichen nach dem Stand der Technik bekannten Methoden erzeugt werden. Beispielsweise kann das Verfahren nach der DE 197 50 794 A1 für die erfindungsgemäßen Dentalglaskeramiken Verwendung finden. Hierbei wird eine Schmelze des Ausgangsglases in der gewünschten Weise geformt und abgekühlt. Das geformte Glasprodukt wird einer Wärmebehandlung unterzogen, um ein als Rohling geformtes Glaskeramikprodukt zu erhalten. Dieses Herstellverfahren liefert sogenannte monolithische Körper.

In einer Variante der Erfindung kann die Herstellung mittels der sogenannten Massivglas/Glaskeramik-Technologie durchgeführt werden:

In Schritt (a) wird eine Schmelze eines Ausgangsglases hergestellt, welches die Komponenten der Glaskeramik enthält. Für diesen Zweck wird eine entsprechende Mischung von geeigneten Ausgangsmaterialien, wie z.B. Carbonaten, Oxiden, Fluoriden und Phosphaten, hergestellt und auf Temperaturen von vorzugsweise 1300 bis 1600 °C für 2 bis 10 Stunden erwärmt. Um einen besonders hohen Grad an Homogenität zu erhalten, kann die erhaltene Glasschmelze in Wasser gegossen werden, um Glaskörner zu bilden und die erhaltenen Glaskörner erneut aufgeschmolzen werden.

In Schritt (b) wird die Schmelze des Ausgangsglases in eine entsprechende Form, z.B. eine Stahlform gegossen und auf Raumtemperatur abgekühlt, um ein Glasprodukt, wie z.B. einen Glasrohling zu erhalten.

Im dritten Verfahrensschritt (c) wird zur Keimbildung und Kristallisation der Glasrohling einer ein- oder mehrstufigen thermischen Behandlung im Temperaturbereich von 700°C bis 1200°C für die Dauer von 30 Minuten bis ca. 6 Stunden unterworfen. Bevorzugt für diese thermischen Behandlungen sind Temperaturen im Bereich von 700°C bis ca. 1000°C und Zeiten von einer bis vier Stunden.

Das Abkühlen wird vorzugsweise in kontrollierter Weise durchgeführt, um eine Entspannung des Glases zu gestatten und Spannungen in der Struktur zu vermeiden, die mit schnellen Temperaturänderungen verbunden sind. In der Regel wird die Schmelze deshalb in auf z.B. 400 °C vorgewärmten Formen gegossen. Anschließend kann der Glasrohling in einem Ofen langsam auf Raumtemperatur abgekühlt werden.

In einer weiteren Variante der Erfindung können Pulver hergestellt werden. So kann zur Herstellung der erfindungsgemäßen Xenotim-Typ-Glaskeramik wie folgt vorgegangen werden:
a) Verarbeitung eines homogenen Rohstoffgemenges, welches die erforderlichen Komponenten beinhaltet, bei Temperaturen von 1200°C bis 1650 °C zu einer Glasschmelze,
b) Fritten der erhaltenen Glasschmelze wird in ein Wasserbad unter Bildung eines Glasgranulates,
c) Zerkleinern des Glasgranulates ggf. zu einem Glaspulver mit einer mittleren Korngröße von 1 bis 500 µm, bezogen auf die Teilchenanzahl, und
d) anschließende Durchführung einer thermischen Behandlung (ein- oder mehrstufig) des Glasgranulates oder des Glaspulvers von 700 bis zu 1200 °C für eine Dauer von 30 Minuten bis 6 Stunden.

In Stufe a) werden zunächst die erforderlichen Einzelkomponenten, wie z.B. Carbonate, Oxide, Fluoride und Phosphate homogen miteinander vermischt und auf die angeführten Temperaturen erwärmt. Hierbei bildet sich das gewünschte Ausgangsglas.

Nachfolgend wird in Stufe b) die erhaltene Glasschmelze durch Eingießen in Wasser abgeschreckt. Es bildet sich ein Glasgranulat. Dieser Herstellungsschritt wird üblicherweise als Fritten bezeichnet.

Ggf. wird in Stufe c) das Glasgranulat nachträglich zerkleinert und hauptsächlich mit üblichen Mühlen auf eine bevorzugte Korngröße gemahlen. Das so hergestellte Glaspulver weist dann eine gewünschte mittlere Korngröße von 1 bis 500 µm, bezogen auf die Teilchenanzahl auf.

Dann wird in Stufe d) das Glasgranulat oder ggf. das Glaspulver einer thermischen Behandlung bei Temperaturen von 700 bis 1200 °C (ein- oder mehrstufig) für eine Dauer von 30 Minuten bis 6 Stunden, vorzugsweise 30 Minuten bis 3 Stunden, unter zogen.

Die in Sinterschritt d) erzeugten Kristalle haben eine vergleichsweise geringe Grösse von etwa 0,1 bis max. 10 µm, bevorzugt zwischen 0,1 und etwa 2 µm. Die Kristalle sind dabei statistisch in der Glaskeramik verteilt und es werden keine bevorzugten Kristall- Wachstumsstellen beobachtet.

Mit den beschriebenen Verfahren können Rohlinge, beispielsweise für die Presstechnik, oder Pulver für die Sintertechnik für die Herstellung eines Fügeverbundwerkstoffes erhalten werden.

Erfindungsgemäß ist es ebenso möglich, die Dentalglaskeramik in Pulverform mit weiteren Dentalglaskeramikpulvern zu verarbeiten. Als solche Dentalglaskeramiken kommen hier beispielsweise Leucit oder Leucit-Fluor-Apatit in Betracht. Hierdurch wird im Endprodukt eine Dental-Mischglaskeramik erhalten.

Bei dieser Verfahrensweise zeigen sich ganz besonders die Vorteile der erfindungsgemäßen Dentalglaskeramik, die sich insbesondere in den hervorragenden Verarbeitungseigenschaften des Xenotim-haltigen Dentalglaspulvers manifestieren. Diese Vorteile sind insbesondere in der Sinterfähigkeit in einem breiten Temperaturbereich bei Aufrechterhaltung der optischen Eigenschaften zu sehen.

Die erfindungsgemässen Dentalglaskeramiken lassen sich in vorstehend beschriebener Weise insbesondere zur Herstellung von Mischglaskeramiken verwenden, insbesondere als sinterbarer Verblendwerkstoff für metallische oder keramische Gerüste.

Erfindungsgemäß lassen sich Glasrohlinge oder Glaskeramikrohlinge herstellen, welche mittels Glaskeramik-Presstechnik (z.B. Empress ® -Technologie der Ivoclar Vivadent AG) zu einem glasigen oder keramischen Formkörper zur Verwendung als Dentalrestauration gepresst werden. Ebenso lässt sich der monolithische Körper durch maschinelle Bearbeitung (z.B. CAD/CAM-Verfahren) zu einem Formkörper zur Verwendung als Dentalrestauration verarbeiten.

Die erfindungsgemäßen Dentalglaskeramiken lassen sich auch als Trübungskomponente, Dentalwerkstoff oder beliebige Dentalrestaurationen verwenden. Der Dentalwerkstoff kann als Beschichtungsmaterial oder Verblendmaterial eingesetzt werden. Die Dentalrestaurationen können eine Krone, eine Brücke, eine Teilkrone, ein Onlay, ein Inlay, ein künstlicher Zahn, ein Stumpfaufbau oder eine Facette sein. Das Beschichtungsmaterial kann zur Beschichtung von metallischen oder/oder keramischen Gerüsten eingesetzt werden.

Im folgenden wird die Erfindung unter Bezugnahme auf die Beispiele näher beschrieben:

**Tabelle 2: Zusammensetzungen und Kristallphasen erfindungsgemässer Glaskeramiken Konzentration in Ma.-%**

| Chem. Komponente/Eigenschaft | 1 | 2 | 3 | 4 | 5 | 6 |
|---|---|---|---|---|---|---|
| SiO₂ | 58.3 | 47.4 | 50.5 | 48.0 | 48.1 | 45.8 |
| Al₂O₃ | 16.6 | 10.6 | 8.3 | 8.1 | 8.2 | 19.2 |
| Y₂O₃ | 2.2 | 9.4 | 18.8 | 18.4 | 18.5 | 26.7 |
| B₂O₃ | 0.3 | 7.9 | | | | |
| Na₂O | 6.4 | 7.1 | 8.3 | 8.1 | 8.2 | |
| K₂O | 9.9 | 10.9 | 7.2 | 7.0 | 7.1 | |
| Li₂O | | | 1.00 | 1.0 | 1.0 | |
| CaO | | 2.3 | | | | |
| ZnO | 1.2 | | | | | |
| P₂O₅ | 1.2 | 3.7 | 3.7 | 7.3 | 7.4 | 8.3 |
| TiO₂ | 1.1 | | | | | |
| ZrO₂ | 2.2 | | 0.8 | 0.8 | 0.8 | |
| F | 0.6 | 0.7 | 0.7 | 0.6 | | |
| CeO₂ | | | 0.7 | 0.7 | 0.7 | |
| Schme Izbed. | 1650°C/2 h | 1550°C/1 h | 1550°C/1.5 h | 1550°C/1.5 h | 1550°C/1.5 h | 1600°C /1h |
| Proben art | Pulver | Pulver | Pulver | Pulver | Pulver | Monolit h |
| T/t (°C, h) | 8h/950°C | 800/1 + 1050/0.5 | 1050/1 | 850/2 | 1050/1 | keine |
| Kristall phasen | YPO₄ * 0.8Z | YP0₄·* 0.8Z, F- Apatit | YPO₄ *·0.8Z | YPO₄ *·0.8Z | YPO₄ *·0.8Z | YPO₄ (Xenoti m) |

| Chem. Komponente/Eigenschaft | 7 | 8 | 9 | 10 |
|---|---|---|---|---|
| SiO₂ | 56.8 | 57.8 | 55.0 | 48.1 |
| Al₂O₃ | 9.3 | 9.5 | 9.0 | 13.2 |
| Y₂O₃ | 6.0 | 6.2 | 12.3 | 5.6 |
| B₂O₃ | - | - | - | 7.2 |
| Na₂O | 9.3 | 9.5 | 9.1 | 4.4 |
| K₂O | 8.0 | 8.2 | 7.8 | 11.1 |
| Li₂O | 1.1 | 1.1 | 1.1 | - |
| CaO | 3.7 | 3.8 | - | - |
| ZnO | - | - | **-** | - |
| P₂O₅ | 4.1 | 2.1 | 4.0 | 7.0 |
| TiO₂ | - | - | - | 1.1 |
| ZrO₂ | 0.9 | 1.0 | 0.9 | 1.7 |
| F | - | - | - | 0.6 |
| CeO₂ | 0.8 | 0.8 | 0.8 | - |
| Schmelzbedingung | 1550°C/1.5 h | 1550°C/1.5 h | 1550°C/1.5h | 1650°C/1h |
| Probenart | Pulver | Pulver | Pulver | Pulver |
| T/t (°C, h) | | | 1000/40 | 850/1 |
| Kristallphasen | | | Na_{3.6}Y_{1.8}(PO₄)₃ | YPO₄ * 0.8Z |

| Chem. Komponente/Eigenschaft | 11 | 12 | 13 | 14 |
|---|---|---|---|---|
| SiO₂ | 41.7 | 46.7 | 56.0 | 49.5 |
| Al₂O₃ | 9.3 | 9.7 | 15.9 | 13.5 |
| Y₂O₃ | 8.3 | | - | 5.7 |
| La₂O₃ | 12.0 | 12.5 | - | - |
| Tb₄O₇ | - | - | 6.2 | - |
| B₂O₃ | 7.0 | 3.2 | 0.3 | 7.5 |
| Na₂O | | 2.8 | 6.2 | 4.6 |
| K₂O | 9.6 | 6.0 | 9.4 | 11.7 |
| Li₂O | - | 1.5 | - | - |
| CaO | - | 2.9 | - | - |
| ZnO | - | - | 1.1 | - |
| P₂O₅ | 5.2 | 5.4 | 1.2 | 7.1 |
| TiO₂ | - | - | 1.0 | - |
| ZrO₂ | - | - | 2.1 | - |
| F | 0.6 | 0.6 | 0.6 | 0.6 |
| Schmelzbedingung | 1550°C/1.5h | 1550°C/1.5h | 1650°C/2h | 1550°C/1.5h |
| Probenart | Pulver | Pulver | Pulver | Pulver |
| T/t (°C, h) | 800/1 + 1050/0.5 | 800/1 + 1050/0.5 | 950/8 | 850°C/1 |
| Kristallphasen | LaPO₄ | LaPO₄ , NaY₉(SiO₄)₆O₂ | TbPO₄ | YPO₄ * 0,8Z |

### Erläuterungen:

Die Beispiele 1 - 5 stellen Xenotim-Typ- Glaskeramiken dar.

Beispiel 6 stellt Xenotim dar, das Glas ist übersättigt und die Kristallisation erfolgt spontan bereits bei Abkühlung der Schmelze.

In den Beispielen 1 - 5 entsteht als Hauptkristallphase ein Xenotim-Typ mit der Formel YPO₄ * 0.8 Z. Diese Kristallphase wurde gemäss der JCPDS- 42-0082 Referenz (Literatur: JACTAW, volume 72, page 1073, (1989) primary reference: Hikichi, Y., Sasaki, T., Murayama, K., Nomura, T., Miyamoto, M.) nachgewiesen. Diese Kristallphase wurde unter dieser Literaturstelle als Hydratphase YPO₄ ·0.8 H₂O dargestellt und könnte nach Hikichi et al. (1989) auch als Rabdophan- Typ bezeichnet werden. Da es sich bei den erfindungsgemässen Glaskeramiken um Hochtemperaturprodukte handelt, ist davon auszugehen, dass ein derartig hoher Wassergehalt im Ausgangsglas der Glaskeramiken nicht vorliegen wird, sodass andere Ionen eingebaut wurden. Fluorid oder Sauerstoff oder andere Ionen , wie sie aus der Isotypie- Beziehung der Apatite bekannt sind, oder aber auch Gitterfehlstellen sind hierbei denkbar. Dies zeigt das Beispiel 5, da dieser Kristalltyp auch ohne Fluorid entsteht, so dass andere Fremdionen in dieses Kristallgitter eingebaut wurden.

Im Beispiel 2 wurde als Nebenphase Fluorapatit gebildet.

Beispiel 9 zeigt die Glaskeramik mit Xenotim-Typ Kristallen, die Na⁺-Ionen in die Kristallstruktur eingebaut haben und der Formel Na_{3.6}Y_{1.8}(PO₄)₃entspricht.

Beispiele 11 bis 13 zeigen die Glaskeramiken mit Monazit-Typ-Kristallen LaPO₄ und TbPO₄ wobei in Beispiel 12 noch eine Nebenphase vom Typ NaY₉(SiO₄)₆O₂ nachgewiesen wurde.

In der nachstehenden Tabelle wurden die Eigenschaften ausgewählter Zusammensetzungen hinsichtlich einiger ihrer Eigenschaften aufgeführt.

**Tabelle 3: Eigenschaften ausgewählter Glaskeramiken**

| Eigenschaft / Glaskeramik gem. Tabelle 1 | 2 | 9 | 11 | 14 |
|---|---|---|---|---|
| Sintertemperatur (°C) | 840 | 960 | 940 | 940 |
| CTE 100- 400 (K⁻¹) | 9.6 * 10⁻⁶ | 10.6 * 10⁻⁶ | 9.5 * 10⁻⁶ | - |
| Chemische Beständigkeit (µg / cm²) | 73 | 27.5 | 33.8 | - |
| Aussehen | n.b. | Milchig getrübt | Milchig opak | Milchig getrübt |
| CR | n.b. | 68.15 | 99.67 | 55.05 |
| L | n.b. | 85.42 | 92.63 | 76.06 |
| a | n.b. | - 0.89 | - 0.16 | - 0.26 |
| b | n.b. | 1.73 | 1.36 | - 0.89 |

Die Buchstaben CR, L, a und b haben folgende Bedeutung:
- CR: Opazität (=Lichtundurchlässigkeit)
- L: Helligkeit
- a: Farbsättigung auf der Rot- Grün- Achse
- b: Farbsättigung auf der Blau- Gelb- Achse

## Patentansprüche

1. Dentalglaskeramik, **dadurch gekennzeichnet, dass** sie wenigstens eine Kristallphase, enthaltend Xenotim oder Monazit oder Gemische hiervon, aufweist, wobei die Kristalle vom Xenotim Typ und/oder Monazit-Typ 0,1 µm bis 10 µm groß sind.

2. Dentalglaskeramik nach Anspruch 1, **dadurch gekennzeichnet, dass** sie Xenotim der chemischen Formel YPO₄ enthält.

3. Dentalglaskeramik nach Anspruch 1, **dadurch gekennzeichnet, dass** sie Xenotim-Typ- Kristalle der chemischen Formel YPO₄ mit einem Anteil von ca. 0,8 Mol Fremdionen enthält oder die Xenotim- Kristalle der Formel ErPO₄ oder LuPO₄ oder TbPO₄ entsprechen.

4. Dentalglaskeramik nach Anspruch 1, **dadurch gekennzeichnet, dass** sie Monazit der Formel CePO₄ enthält.

5. Dentalglaskeramik nach Anspruch 1, **dadurch gekennzeichnet, dass** sie Kristalle vom Monazit-Typ, wie LaPO₄, DyPO₄, GdPO₄ und TbPO₄ enthält.

6. Dentalglaskeramik nach einem der vorhergehenden Ansprüche, **dadurch gekennzeichnet, dass** die Kristalle zwischen 0,1 µm und ca. 2 µm groß sind.

7. Dentalglaskeramik nach einem der vorhergehenden Ansprüche, **dadurch gekennzeichnet, dass** sie wenigstens SiO₂, Al₂O₃, R-Oxide und P₂O₅ enthält, wobei R für Seltenerden steht.

8. Dentalglaskeramik nach Anspruch 7, **dadurch gekennzeichnet, dass** die R-Oxide ausgewählt sind aus der Gruppe Y₂O₃, Er₂O₃, Lu₂O₃, La₂O₃, CeO₂ und Tb₄O₇.

9. Dentalglaskeramik nach einem der vorhergehenden Ansprüche, **dadurch gekennzeichnet, dass** sie im wesentlichen die Zusammensetzung aus
| | |
|---|---|
| SiO₂ | 40 - 65 Ma.-% |
| Al₂O₃ | 5 - 25 Ma.-% |
| R-Oxide | 0.5 - 30 Ma.-% |
| P₂O₅ | 0.5 -10 Ma.-% |
aufweist und die Summe der Einzelkomponenten 100 Ma.-% beträgt, wobei R für Seltenerden steht.

10. Dentalglaskeramik nach Anspruch 9, **dadurch gekennzeichnet, dass** sie als weitere Zusatzkomponenten
| | |
|---|---|
| Fluorid | 0 - 5 Ma.-% |
| Zusatzoxide | 5 - 30 Ma.-% |
aufweist, wobei die Zusatzoxide ausgewählt sind aus der Gruppe Li₂O, Na₂O, K₂O, CaO, MgO, ZnO, B₂O₃, TiO₂ und/oder ZrO₂.

11. Dentalglaskeramik nach einem der vorhergehenden Ansprüche, **dadurch gekennzeichnet, dass** sie bevorzugt 10 bis 30 Ma.-% an Zusatzoxiden enthält.

12. Dentalglaskeramik nach einem der vorhergehenden Ansprüche, **dadurch gekennzeichnet, dass** sie bevorzugt zusammengesetzt ist aus
| | |
|---|---|
| SiO₂ | 45 - 60 Ma.-% |
| Al₂O₃ | 8 - 20 Ma.-% |
| Y₂O₃ | 5 - 30 Ma.-% |
| P₂O₅ | 1 - 10 Ma.-% |
| B₂O₃ | 0 - 10 Ma.-% |
| Na₂O | 0 - 15 Ma.-% |
| K₂O | 0 - 15 Ma.-% |
| Li₂O | 0 - 5 Ma.-% |
| CaO | 0 - 10 Ma.-% |
| Fluorid | 0 - 5 Ma.-% |
aufweist und die Summe der Komponenten stets 100 Ma.-% beträgt.

13. Dentalgiaskeramik nach einem der vorhergehenden Ansprüche, **dadurch gekennzeichnet, dass** sie als weitere Kristalle Apatite oder Leucite aufweist.

14. Verfahren zur Herstellung der Dentalglaskeramik nach einem der vorhergehenden Ansprüche, **dadurch gekennzeichnet, dass** man
(a) die zur Herstellung der Glaskeramik erforderlichen Komponenten mischt und zu einem Glas aufschmilzt,
(b) die Glasschmelze aus Schritt (a) durch Fritten in ein Glasgranulat überführt,
(c) ggf. das Glasgranulat zu einem Pulver mit einer mittleren Korngröße von 1 bis 500 µm (bezogen auf die Teilchenzahl) zerkleinert, und
(d) das Glasgranulat aus Schritt (b) oder das Glaspulver aus Schritt (c) anschließend einer ein- oder mehrstufigen thermischen Behandlung im Temperaturbereich von 700°C bis 1200°C für die Dauer von ca. 30 Minuten bis ca. 6 Stunden unterwirft.

15. Verfahren zur Herstellung einer Mischglaskeramik, **dadurch gekennzeichnet, dass** man eine Xenotim- und/oder Monazit-Kristalle enthaltende Dentalglaskeramik in Pulverform nach Anspruch 14 mit weiteren Gläsern oder Glaskeramiken in Pulverform mischt, wobei die weiteren Gläser vorzugsweise ausgewählt sind aus der Gruppe der Opalgläser, der Alkalisilicatgläser und/oder der bei niedriger Temperatur sinternden Kalium-Zink-Silicat-Gläser sowie die weiteren Glaskeramiken vorzugsweise ausgewählt sind aus der Gruppe der tiefsinternden Apatitglaskeramiken, der transluzenten Apatitglaskeramiken, der sinterbaren Lithiumsilicatglaskeramiken, der ZrO₂-SiO₂- Glaskeramiken und/oder der Leucit enthaltenden Phosphosilicatglaskeramiken.

16. Verfahren zur Herstellung der Dentalglaskeramik nach einem der Ansprüche 1 bis 13, **dadurch gekennzeichnet, dass** man
(a) die zur Herstellung der Glaskeramik erforderlichen Komponenten mischt und zu einem Glas aufschmilzt,
(b) die Glasschmelze aus Schritt (a) in eine, vorzugsweise vorgewärmte, Form gießt und kontrolliert auf Raumtemperatur abgekühlt, um einen geformten Glasrohling zu erhalten und
(c) den Glasrohling einer ein- oder mehrstufigen thermischen Behandlung im Temperaturbereich von 700°C und 1200°C für die Dauer von 30 Minuten bis ca. 6 Stunden unterwirft.

17. Verwendung der Dentalglaskeramiken nach einem der Ansprüche 1 bis 13 oder einer Mischglaskeramik gemäß Anspruch 15 als Trübungskomponente, Dentalwerkstoff, zur Herstellung eines Dentalwerkstoffs oder einer Dentalrestauration.

18. Verwendung der Dentalglaskeramik oder der Mischglaskeramik nach Anspruch 17, **dadurch gekennzeichnet, dass** der Dentalwerkstoff ein Beschichtungsmaterial oder ein Verblendmaterial ist.

19. Verwendung der Dentalglaskeramik oder der Mischglaskeramik nach Anspruch 17, **dadurch gekennzeichnet, dass** die Dentalrestauration eine Krone, Brücke, Teilkrone, ein Onlay, ein Inlay, ein künstlicher Zahn, ein Stumpfaufbau oder eine Facette ist.

20. Verwendung der Dentalglaskeramik oder der Mischglaskeramik nach Anspruch 18, **dadurch gekennzeichnet, dass** das Beschichtungsmaterial für die Beschichtung von metallischen und/oder keramischen Gerüsten verwendet wird.

21. Verwendung des nach Anspruch 16 erhaltenen Glasrohlings oder des Glaskeramikrohlings als Dentalrestauration, **dadurch gekennzeichnet, dass** der monolithische Körper mittels der Keramik- Presstechnik zu einem glasigen oder keramischen Formkörper gepresst wird.

22. Verwendung des nach Anspruch 16 erhaltenen Glasrohlings oder des Glaskeramikrohlings als Dentalrestauration, **dadurch gekennzeichnet, dass** der monolithische Körper durch maschinelle Bearbeitung zu einem Formkörper bearbeitet wird.

## Claims

1. Dental glass-ceramic, **characterized in that** it comprises at least one crystal phase containing xenotime or monazite or mixtures thereof, where the crystals of the xenotime type and/or monazite type have a size of from 0.1 µm to 10 µm.

2. Dental glass-ceramic according to Claim 1, **characterized in that** it contains xenotime of the chemical formula YPO₄.

3. Dental glass-ceramic according to Claim 1, **characterized in that** it contains xenotime-type crystals of the chemical formula YPO₄ having a proportion of about 0.8 mol of foreign ions or the xenotime crystals correspond to the formula ErPO₄ or LuPO₄ or TbPO₄.

4. Dental glass-ceramic according to Claim 1, **characterized in that** it contains monazite of the formula CePO₄.

5. Dental glass-ceramic according to Claim 1, **characterized in that** it contains crystals of the monazite type such as LaPO₄, DyPO₄, GdPO₄ and TbPO₄.

6. Dental glass-ceramic according any of the preceding claims, **characterized in that** the crystals have a size of from 0.1 µm to about 2 µm.

7. Dental glass-ceramic according to any of the preceding claims, **characterized in that** it contains at least SiO₂, Al₂O₃, R oxides and P₂O₅, where R is a rare earth.

8. Dental glass-ceramic according to Claim 7, **characterized in that** the R oxides are selected from the group consisting of Y₂O₃, Er₂O₃, Lu₂O₃, La₂O₃, CeO₂ and Tb₄O₇.

9. Dental glass-ceramic according to any of the preceding claims, **characterized in that** it has essentially the composition comprising
| | |
|---|---|
| SiO₂ | 40 - 65% by mass |
| Al₂O₃ | 5 - 25% by mass |
| R oxides | 0.5 - 30% by mass |
| P₂O₅ | 0.5 - 10% by mass |
and the sum of the individual components is 100% by mass, where R is a rare earth.

10. Dental glass-ceramic according to Claim 9, **characterized in that** it has
| | |
|---|---|
| fluoride | 0 - 5% by mass |
| additional oxides | 5 - 30% by mass |
as further additional components, where the additional oxides are selected from the group consisting of Li₂O, Na₂O, K₂O, CaO, MgO, ZnO, B₂O₃, TiO₂ and ZrO₂.

11. Dental glass-ceramic according to any of the preceding claims, **characterized in that** it preferably contains from 10 to 30% by mass of additional oxides.

12. Dental glass-ceramic according to any of the preceding claims, **characterized in that** it is preferably composed of
| | |
|---|---|
| SiO₂ | 45 - 60% by mass |
| Al₂O₃ | 8 - 20% by mass |
| Y₂O₃ | 5 - 30% by mass |
| P₂O₅ | 1 - 10% by mass |
| B₂O₃ | 0 - 10% by mass |
| Na₂O | 0 - 15% by mass |
| K₂O | 0 - 15% by mass |
| Li₂O | 0 - 5% by mass |
| CaO | 0 - 10% by mass |
| fluoride | 0 - 5% by mass |
and the sum of the components is always 100% by mass.

13. Dental glass-ceramic according to any of the preceding claims, **characterized in that** it comprises apatite or leucite as further crystals.

14. Process for producing the dental glass-ceramic according to any of the preceding claims, **characterized in that**
(a) the components required for producing the glass-ceramic are mixed and melted to form a glass,
(b) the glass melt from step (a) is converted by fritting into glass granules,
(c) if appropriate, the glass granules are comminuted to give a powder having an average particle size of from 1 to 500 µm (based on the number of particles) and
(d) the glass granules from step (b) or the glass powder from step (c) are/is subsequently subjected to a single-stage or multistage thermal treatment in the temperature range from 700°C to 1200°C for a period of from about 30 minutes to about 6 hours.

15. Process for producing a mixed glass-ceramic, **characterized in that** a pulverulent dental glass-ceramic containing xenotime and/or monazite crystals according to Claim 14 is mixed with further pulverulent glasses or glass-ceramics, where the further glasses are preferably selected from the group consisting of opal glasses, alkali metal silicate glasses and/or potassium zinc silicate glasses which sinter at a low temperature and the further glass-ceramics are preferably selected from the group consisting of low-sintering apatite glass-ceramics, translucent apatite glass-ceramics, sinterable lithium silicate glass-ceramics, ZrO₂-SiO₂ glass-ceramics and/or leucite-containing phosphosilicate glass-ceramics.

16. Process for producing the dental glass-ceramic according to any of Claims 1 to 13, **characterized in that**
(a) the components required for producing the glass-ceramic are mixed and melted to form a glass,
(b) the glass melt from step (a) is poured into a preferably pre-heated mold and is cooled in a controlled manner to room temperature in order to obtain a shaped glass blank and
(c) the glass blank is subjected to a single-stage or multistage thermal treatment in the temperature range from 700°C to 1200°C for a period of from 30 minutes to about 6 hours.

17. Use of the dental glass-ceramics according to any of Claims 1 to 13 or a mixed glass-ceramic according to Claim 15 as opacifying component, dental material, for producing a dental material or a dental restoration.

18. Use of the dental glass-ceramic or the mixed glass-ceramic according to Claim 17, **characterized in that** the dental material is a coating material or a facing material.

19. Use of the dental glass-ceramic or the mixed glass-ceramic according to Claim 17, **characterized in that** the dental restoration is a crown, bridge, part crown, an onlay, an inlay, an artificial tooth, a stump buildup or a facing.

20. Use of the dental glass-ceramic or the mixed glass-ceramic according to Claim 18, **characterized in that** the coating material is used for coating metal and/or ceramic substrates.

21. Use of the glass blank obtained according to Claim 16 or the glass-ceramic blank as dental restoration, **characterized in that** the monolithic body is pressed by means of the ceramic pressing technique to produce a vitreous or ceramic shaped body.

22. Use of the glass blank obtained according to Claim 16 or the glass-ceramic blank as dental restoration, **characterized in that** the monolithic body is machined to produce a shaped body.

## Revendications

1. Vitrocéramique dentaire, **caractérisée en ce qu'**elle présente au moins une phase cristalline contenant du xénotime ou de la monazite ou des mélanges de ceux-ci, les cristaux du type xénotime et/ou du type monazite présentant une taille de 0,1 µm à 10 µm.

2. Vitrocéramique dentaire selon la revendication 1, **caractérisée en ce qu'**elle contient du xénotime de formule chimique YPO₄.

3. Vitrocéramique dentaire selon la revendication 1, **caractérisée en ce qu'**elle contient des cristaux de type xénotime de formule chimique YPO₄ présentant une proportion d'environ 0,8 mole d'ions étrangers ou les cristaux de xénotime correspondent à la formule ErPO₄ ou LuPO₄ ou TbPO₄.

4. Vitrocéramique dentaire selon la revendication 1, **caractérisée en ce qu'**elle contient de la monazite de formule CePO₄.

5. Vitrocéramique dentaire selon la revendication 1, **caractérisée en ce qu'**elle contient des cristaux de type monazite, tels que LaPO₄, DyPO₄, GdPO₄ et TbPO₄.

6. Vitrocéramique dentaire selon l'une quelconque des revendications précédentes, **caractérisée en ce que** les cristaux présentent une taille entre 0,1 µm et environ 2 µm.

7. Vitrocéramique dentaire selon l'une quelconque des revendications précédentes, **caractérisée en ce qu'**elle contient au moins du SiO₂, de l'Al₂O₃, des oxydes de R et du P₂O₅, où R représente des terres rares.

8. Vitrocéramique dentaire selon la revendication 7, **caractérisée en ce que** les oxydes de R sont choisis dans le groupe formé par Y₂O₃, Er₂O₃, Lu₂O₃, La₂O₃, CeO₂ et Tb₄O₇.

9. Vitrocéramique dentaire selon l'une quelconque des revendications précédentes, **caractérisée en ce qu'**elle présente essentiellement la composition constituée par
| | |
|---|---|
| SiO₂ | 40-65% en masse |
| Al₂O₃ | 5-25% en masse |
| oxydes de R | 0,5-30% en masse |
| P₂O₅ | 0,5-10% en masse |
et la somme des différents composants vaut 100% en masse, où R représente des terres rares.

10. Vitrocéramique dentaire selon la revendication 9, **caractérisée en ce qu'**elle contient comme autres composants supplémentaires
fluorure 0-5% en masse
oxydes supplémentaires 5-30% en masse
où les oxydes supplémentaires sont choisis dans le groupe formé par Li₂O, Na₂O, K₂O, CaO, MgO, ZnO, B₂O₃, TiO₂ et/ou ZrO₂.

11. Vitrocéramique dentaire selon l'une quelconque des revendications précédentes, **caractérisée en ce qu'**elle contient de préférence 10 à 30% en masse d'oxydes supplémentaires.

12. Vitrocéramique dentaire selon l'une quelconque des revendications précédentes, **caractérisée en ce qu'**elle est de préférence composée de
| | |
|---|---|
| SiO₂ | 45-60% en masse |
| Al₂O₃ | 8-20% en masse |
| Y₂O₃ | 5-30% en masse |
| P₂O₅ | 1-10% en masse |
| B₂O₃ | 0-10% en masse |
| Na₂O | 0-15% en masse |
| K₂O | 0-15% en masse |
| Li₂O | 0-5% en masse |
| CaO | 0-10% en masse |
| fluorure | 0-5% en masse |
et la somme des composants est toujours égale à 100% en poids.

13. Vitrocéramique dentaire selon l'une quelconque des revendications précédentes, **caractérisée en ce qu'**elle présente comme autres cristaux de l'apatite ou de la leucite.

14. Procédé pour la préparation de la vitrocéramique dentaire selon l'une quelconque des revendications précédentes, **caractérisé en ce que**
(a) on mélange les composants nécessaires pour la préparation de la vitrocéramique et on les fait fondre en un verre,
(b) on transforme la masse fondue de verre de l'étape (a) par frittage en un granulat de verre,
(c) le cas échéant on broie le granulat de verre en une poudre présentant une grosseur moyenne de 1 à 500 µm (par rapport au nombre de particules), et
(d) on soumet le granulat de verre de l'étape (b) ou la poudre de verre de l'étape (c) ensuite à un traitement thermique en une ou plusieurs étapes, dans la plage de température de 700°C à 1200°C pour la durée d'environ 30 minutes à environ 6 heures.

15. Procédé pour la préparation d'une vitrocéramique mixte, **caractérisé en ce qu'**on mélange une vitrocéramique dentaire contenant des cristaux de xénotime et/ou de monazite sous forme de poudre selon la revendication 14 avec d'autres verres ou vitrocéramiques sous forme de poudre, où les autres verres sont de préférence choisis dans le groupe des opalines, des verres à base de silicate de métal alcalin et/ou des verres à base de silicate de potassium-zinc frittant à basse température et les autres vitrocéramiques sont de préférence choisies dans le groupe des vitrocéramiques à base d'apatite frittant à basse température, des vitrocéramiques à base d'apatite translucides, des vitrocéramiques à base de silicate de lithium frittables, des vitrocéramiques à base de ZrO₂-SiO₂ et/ou des vitrocéramiques à base de phosphosilicates contenant de la leucite.

16. Procédé pour la préparation de la vitrocéramique dentaire selon l'une quelconque des revendications 1 à 13, **caractérisé en ce que**
(a) on mélange les composants nécessaires pour la préparation de la vitrocéramique et on les fait fondre en un verre,
(b) on coule la masse fondue de verre de l'étape (a) dans un moule, de préférence préchauffée et on la refroidit de manière contrôlée à température ambiante pour obtenir un corps cru de verre moulé et
(c) on soumet le corps cru de verre à un traitement thermique en une ou plusieurs étapes, dans la plage de température de 700°C à 1200°C pour la durée d'environ 30 minutes à environ 6 heures.

17. Utilisation des vitrocéramiques dentaires selon l'une quelconque des revendications 1 à 13 ou d'une vitrocéramique mixte selon la revendication 15 comme composant de trouble, matériau dentaire, pour la réalisation d'un matériau dentaire ou d'une restauration dentaire.

18. Utilisation de la vitrocéramique dentaire ou de la vitrocéramique mixte selon la revendication 17, **caractérisée en ce que** le matériau dentaire est un matériau de revêtement ou un matériau de recouvrement.

19. Utilisation de la vitrocéramique dentaire ou de la vitrocéramique mixte selon la revendication 17, **caractérisée en ce que** la restauration dentaire est une couronne, un bridge, une couronne partielle, un onlay, un inlay, une dent artificielle, une construction sur moignon ou une facette.

20. Utilisation de la vitrocéramique dentaire ou de la vitrocéramique mixte selon la revendication 18, **caractérisée en ce que** le matériau de revêtement est utilisé pour le revêtement de structures métalliques et/ou céramiques.

21. Utilisation du corps cru de verre obtenu selon la revendication 16 ou du corps cru de vitrocéramique comme restauration dentaire, **caractérisée en ce que** le corps monolithique est pressé au moyen de la technique de pressage de céramique en un corps façonné vitreux ou céramique.

22. Utilisation du corps cru de verre obtenu selon la revendication 16 ou du corps cru de vitrocéramique comme restauration dentaire, **caractérisée en ce que** le corps monolithique est usiné par usinage machinal en corps façonné.
